(19) Europäisches Patentamt
European Patent Office
Office européen des brevets

(11) Publication number: **0 115 961**
**A2**

(12) **EUROPEAN PATENT APPLICATION**

(21) Application number: **84300673.5**

(22) Date of filing: **03.02.84**

(51) Int. Cl.[3]: **C 07 D 223/16**, C 07 C 123/00 // C07C119/20

(30) Priority: **04.02.83 US 463796**

(43) Date of publication of application: **15.08.84 Bulletin 84/33**

(84) Designated Contracting States: **AT BE CH DE FR GB IT LI LU NL SE**

(71) Applicant: **ELI LILLY AND COMPANY, 307, East McCarty Street, Indianapolis Indiana 46285 (US)**

(72) Inventor: **Robey, Roger Lewis, 1785 Brer Rabbit Drive, Greenwood Indiana 46142 (US)**

(74) Representative: **Crowther, Terence Roger et al, Erl Wood Manor, Windlesham Surrey GU20 6PH (GB)**

(54) **Process for preparing benzazepines.**

(57) This invention relates to a novel process for preparing 2-amino-8-alkoxybenzazepines. The process involves cyclizing an intermediate alkoxybenzene-ethanimidate with a catalytic amount of one or more acids, optionally in the presence of one or more non-reactive solvents.

# PROCESS FOR PREPARING BENZAZEPINES

The present invention relates to a new and improved process and intermediates for preparing certain benzazepines which are taught to have antihypertensive activity in copending application Serial No. X-4826, filed at even date herewith.

Several methods of preparing 2-amino-1H-3-benzazepines are found in the literature. In J. Het. Chem., 2, 26 (1965), 2-amino-1H-3-benzazepine was prepared from the 4-bromo precursor. The bromo intermediate was prepared from the corresponding o-phenylenediacetonitrile by treatment with hydrogen bromide in acetic acid. This approach is limited to those compounds for which the o-di(cyanomethylene)phenyl precursors can be prepared. These dicyanomethylene intermediates may be prepared by reacting cyanide ion with the respective 1,2-di(halomethyl)benzenes. The di(halomethyl)benzenes, otherwise known as α,α'-dihalo-o-xylenes, may be prepared by haloalkylation of benzenes, free radical halogenation of o-xylenes, and similar methods known in the art.

Other methods use a phenylacetic acid derivative as starting material. The phenylacetic acid may be condensed with an aminoacetaldehyde dialkyl acetal by methods known in the art and then cyclized to the corresponding lactam in the presence of an acidic cyclization reagent. The cyclic lactam intermediate may be transformed into the 2-aminobenzazepine by first transforming the lactam into the corresponding thiolactam or 2-alkoxy-1H-3-benzazepine intermediates.

These latter intermediates may then be transformed to the desired 2-aminobenzazepines on treatment with the appropriate amine.

All of the above transformations involve long and laborious multistep syntheses which involve starting materials which are sometimes difficult to prepare or require reagents which are expensive, difficult, or dangerous to use. For instance, the conversion of the lactam to thiolactam intermediates in the above sequence employs phosphorous pentasulfide which creates odor, waste stream, and purification problems. Thus, a simple, high yielding, and inexpensive process of preparing the useful 2-amino-1H-3-benzazepines is desirable. Especially desirable is a process which uses easy to obtain starting materials and reagents. These improved process features are provided by the present invention.

The literature teaches that when an imino ether (otherwise known as an imidate) is reacted with an aminoacetaldehyde dialkyl acetal, the corresponding N-(2,2-dialkoxyethyl)substituted amidine is formed. The treatment of this amidine with acid provides the respective imidazole derivative. See generally D. G. Neilson, The Chemistry of Amidines and Imidates, S. Patai, ed., John Wiley and Sons, 1975, p. 443. This reaction sequence was recently used to prepare 2-benzylimidazole derivatives from a corresponding alkylphenylacetamidate. (See British Patent No. 1,336,228.) Thus, the literature teaches away from the instant process.

In contrast to this literature, the current invention provides an improved process for preparing certain substituted 2-amino-1H-3-benzazepines from N-(2,2-dialkoxyethyl)-substituted benzylamidines and novel intermediate compounds therefor.

This invention provides a process of preparing compounds of formula I

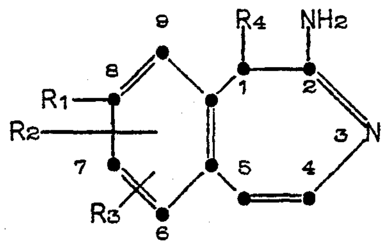 I

or pharmaceutically acceptable salts thereof, wherein:

$R_1$ is $C_1$-$C_3$ alkoxy;

$R_2$ and $R_3$ are each independently hydrogen, $C_1$-$C_3$ alkoxy, or $C_1$-$C_3$ alkyl; and

$R_4$ is hydrogen or methyl;

which comprises reacting a compound of formula II

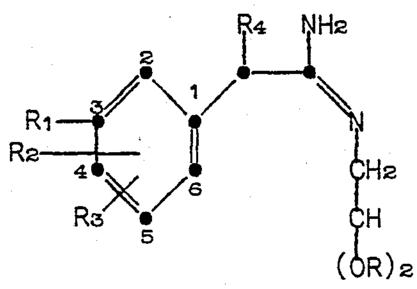 II

or a salt thereof, wherein $R_1$, $R_2$, $R_3$ and $R_4$ are defined as above and R is $C_1$-$C_6$ alkyl, with at least a catalytic amount of one or more acids, optionally in the presence of one or more non-reactive solvents.

The novel intermediate compounds of formula II above are also claimed as a part of the invention. As will be appreciated by those skilled in the art, the substituted amidine of structure II may in fact also exist as the tautomer II'

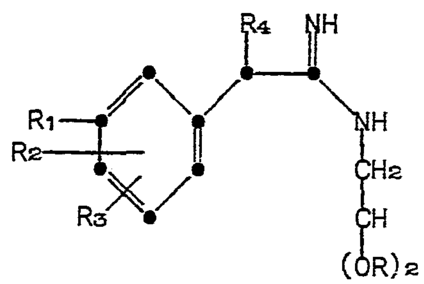

II'

Either tautomeric form or a mixture of forms is claimed as part of this invention.

The term "$C_1$-$C_6$ alkyl" refers to the straight and branched aliphatic radicals of one to six carbon atoms such as methyl, ethyl, isopropyl, n-propyl, n-butyl, tert-butyl, pentyl, isopentyl, hexyl, and the like, and includes within its definition the term "$C_1$-$C_3$ alkyl". The term "$C_1$-$C_3$ alkoxy" refers to methoxy, ethoxy, n-propoxy and isopropoxy.

The preferred compounds of formula I prepared from the new intermediates of formula II are those wherein $R_4$ is hydrogen and $R_2$ is $C_1$-$C_3$ alkoxy, especially

where $R_1$ and $R_2$ are each methoxy, most preferably 2-amino-7,8-dimethoxy-1H-3-benzazepine.

The compounds of formula I are prepared by the acid catalyzed cyclization of the corresponding intermediates of formula II or an acid addition salt thereof. Especially preferred acid addition salts of compounds of formula II are mineral acid salts, especially the hydrochloric acid salt. Any of a number of acids may be used to effect the desired cyclization including one or more of the mineral acids, alkanesulfonic acids, aromatic sulfonic acids, perfluoronated carboxylic acids, such as trifluoroacetic acid, and other strong organic acids.

The reaction may be performed in the presence or the absence of one or more non-reactive solvents. Virtually any solvent can be employed with the restriction that it should be compatible with acidic medium and the compounds of formulae I and II. Such solvents include water, $C_2$-$C_{20}$ alkanoic acids, especially $C_2$-$C_6$ alkanoic acids, such as acetic acid or propionic acid, $C_3$-$C_{20}$ alkenoic acids, especially $C_3$-$C_6$ alkenoic acids, $C_3$-$C_6$ alkynoic acids, ketones such as $C_1$-$C_6$ dialkyl ketones for example acetone, esters such as ethyl acetate, hexyl acetate, ethyl propionate or propyl acetate, aromatic or aliphatic halides such as methylene chloride, chlorobenzene, or chloroform, hydrocarbons such as hexane or toluene, ethers such as tetrahydrofuran or 1,2-dimethoxyethane, and others, or suitable combinations thereof, such as acetic acid and tetrahydrofuran or acetic acid and 1,2-dimethoxyethane.

Preferred reaction media are gaseous hydrogen chloride in acetic acid, aqueous hydrochloric acid, gaseous hydrogen chloride in a mixture of acetic acid and 1,2-dimethoxyethane, and methanesulfonic acid in acetic acid.

The reaction may be performed at a temperature range of about 0°C. to reflux, especially from about 0°C to about 100°C. The preferred reaction temperature range is about 20-60°C.

The amount of acid used may vary from a catalytic amount, preferably in the presence of a solvent, to a tremendous excess where the acid also serves as the solvent. For economic reasons, the former conditions are preferred, especially where an inexpensive and/or recoverable solvent is employed. The term "catalytic amount" of acid means the minimum amount of acid sufficient to cause the cyclization of a compound of formula II to a compound of formula I. In the case of an acid addition salt of formula II this amount is usually approximately a molar equivalent of acid, although depending upon the acid, the solvent, and other variables (i.e., concentration, temperature, and others), considerably more or less acid may be required to constitute a "catalytic amount". In the preferred case of hydrogen chloride in acetic acid being employed for the cyclization, two molar equivalents of hydrogen chloride are required to convert the acid addition salt of formula II to the corresponding compound of formula I, whereas only a single equivalent of hydrogen chloride

gives the undesired imidazole derivative as taught in the literature.

The intermediates of formula II may be prepared by methods known in the art according to the following scheme:

Scheme I

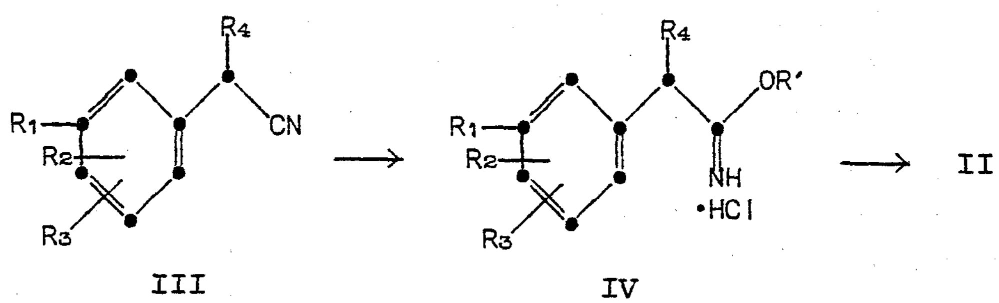

III IV

where $R_1$, $R_2$, $R_3$, and $R_4$ are the same as described hereinabove and R' is $C_1$-$C_6$ alkyl.

Imidate intermediate of formula IV may be prepared by treating the corresponding nitrile intermediate of formula III with hydrogen chloride gas dissolved in the alcohol R'OH. This transformation is otherwise known as the Pinner reaction. This imidate intermediate is transformed to amidine of formula II by treating with an aminoacetaldehyde dialkyl acetal, preferably the dimethyl or diethyl acetal, in a nonreactive solvent such as an alcohol, an ether such as tetrahydrofuran or 1,2-dimethoxyethane, and others. The reaction is carried out at temperatures from about 0°C. up to the reflux temperature of the reaction mixture. A temperature of about ambient temperature to about 60°C. is preferred. In the preferred conditions, tetrahydrofuran or dimethoxyethane is the solvent for the transformation of a compound of formula IV to a compound of formula II. Intermediates of formula II may be isolated or preferably is used in situ for the conversion to a compound of formula I by the subsequent addition of the acid catalyst and optionally one or more additional solvents, with acetic acid being the preferred solvent, alone or in combination with other compatible solvents.

The nitrile intermediates of formula III are either commercially available, are known in the art, or can be prepared from methods known in the art. The cyano intermediate of formula III may be prepared from the respective benzyl halide by reacting with cyanide ion. The corresponding benzyl halide can be prepared by haloalkylation of benzenes, free radical halogenation of toluenes, and similar methods known in the art.

In an effort to more fully illustrate the operation of this invention, the following detailed examples are provided. The examples are illustrative only and are not intended to limit the scope of the invention.

## Example 1

2-Amino-7,8-dimethoxy-1H-3-benzazepine hydrochloride

(A) Ethyl 3,4-dimethoxybenzeneethanimidate hydrochloride

To a slurry of 17.7 g. of 3,4-dimethoxyphenylacetonitrile in 75 ml. of dry ethanol previously chilled to 5°C. by means of an external ice bath was bubbled dry

hydrogen chloride gas. The gas was introduced at such a rate so as to maintain the temperature below 15°C. After 2.5 hours of adding the gas, all of the slurry had dissolved. Gas addition was stopped and the reaction was allowed to warm to room temperature. The reaction mixture was then allowed to stir at room temperature for about three days. The solution was evaporated *in vacuo*. To the resulting syrup was added about 100 ml. of diethyl ether. The resulting white solid was recovered by filtration affording 23 g. of the title product, m.p. about 110-111°C. (literature m.p. 114.5-115°C., *J. Chem. Soc.*, 2157 (1959)).

(B) N-(2,2-dimethoxyethyl)-3,4-dimethoxybenzeneethanimidamide hydrochloride

To a solution of 1.21 g. of aminoacetaldehyde dimethyl acetal in 12 ml. of dry ethanol were added 3.0 g. of ethyl 3,4-dimethoxybenzeneethanimidate hydrochloride (prepared in Part A). The reaction mixture stirred at room temperature for two hours. The reaction mixture was evaporated *in vacuo* affording 3.96 g. of the title product as a foam.

(C) 2-Amino-7,8-dimethoxy-1H-3-benzazepine hydrochloride

To a solution of 4.77 g. of N-(2,2-dimethoxyethyl)-3,4-dimethoxybenzeneethanimidamide hydrochloride (prepared in Part B) in 12 ml. of acetic acid was bubbled dry hydrogen chloride gas. After the solution became saturated, gas addition was stopped and the reaction was stirred overnight at about 50°C. After an

hour of stirring, an additional 10 ml. of acetic acid was added to facilitate stirring of the suspension that formed. The solution was cooled to room temperature and the precipitate was filtered, washed with cold acetic acid, and dried affording 1.73 g. of the title product, m.p. about 279-281°C.

## Example 2

N-(2,2-diethoxyethyl)-3,4-dimethoxybenzeneethanimidamide hydrochloride

The title compound was prepared following the procedure of Example 1B using aminoacetaldehyde diethyl acetal in place of the dimethyl acetal.

## Example 3

Alternate procedure for the preparation of 2-amino-7,8-dimethoxy-1H-3-benzazepine hydrochloride

A solution of 3.0 g. of ethyl 3,4-dimethoxybenzeneethanimidate hydrochloride and 1.21 g. of aminoacetaldehyde dimethyl acetal in 12 ml. of 1,2-dimethoxyethane was stirred overnight at room temperature. Twelve milliliters of acetic acid were added and dry hydrogen chloride gas was bubbled into the solution. After the solution was saturated, gas addition was stopped and the solution was heated to about 50°C. for about four hours. The reaction was then stirred overnight at room temperature. The title product (2.3 g.) was recovered by filtration, m.p. about 280-281°C.

A yield of 2.25 g. was recovered when the identical process was performed using tetrahydrofuran in place of dimethoxyethane.

Example 4

2-Amino-6,7,8-trimethoxy-1H-3-benzazepine hydrochloride

3,4,5-Trimethoxyphenylacetonitrile was converted to the corresponding ethyl imidate following the procedure of Example 1A. Two grams of the imidate intermediate were suspended in 10 ml. of dry tetrahydrofuran at about 5°C. A solution of 0.72 g. of aminoacetaldehyde dimethyl acetal in 1 ml. of tetrahydrofuran was added and the reaction was stirred overnight at room temperature. The reaction was evaporated *in vacuo* and 10 ml. of acetic acid were added. Methanesulfonic acid (0.85 g.) was added and the reaction was stirred overnight at room temperature. Filtration of the resulting precipitate afforded 0.50 g. of the title product, m.p. about 274-275°C. The filtrate was poured into ice and 50% sodium hydroxide. Filtration of the resulting precipitate provided 0.80 g. of the free base of the title product, m.p. about 155-162°C.

## Example 5

2-Amino-8-methoxy-1H-3-benzazepine hydrochloride

Following the procedure of Example 1A, 3-methoxyphenylacetonitrile was transformed into the corresponding imidate. The imidate intermediate was reacted with aminoacetaldehyde dimethyl acetal and then methanesulfonic acid following the procedure of Example 4. The acid cyclization reaction was worked up by pouring into ice/25% sodium hydroxide. The free base of the title product was recovered by filtration. The title product was prepared by suspending the free base in acetic acid, adding concentrated hydrochloric acid, and filtering the crystals that formed after standing at room temperature for two days, m.p. decomposition about 252-254°C. The NMR and infrared spectra were consistent with the desired structure.

## Example 6

2-Amino-7-methoxy-8-ethoxy-1H-3-benzazepine hydrochloride

The title product was prepared from 3-ethoxy-4-methoxyphenylacetonitrile following the procedures of Examples 1, and 2, m.p. about 255-256°C.

Analysis: $C_{13}H_{16}N_2O_2 \cdot HCl$;

Calc.: C, 58.10; H, 6.38; N, 10.42; Cl, 13.19;

Found : C, 58.32; H, 6.50; N, 10.60; Cl, 12.92.

## Example 7

Alternate procedure for the preparation of 2-amino-7,8-dimethoxy-1H-3-benzazepine hydrochloride

A solution of 3.8 g. of N-(2,2-dimethoxyethyl)-3,4-dimethoxybenzeneethanimidamide hydrochloride, 3 ml. of concentrated hydrochloric acid, and 12 ml. of acetic acid was stirred overnight at room temperature. To the mixture was added acetonitrile and the product filtered to yield 1.2 g., m.p. 279-281°C.

## Example 8

Alternate procedure for the preparation of 2-amino-7,8-dimethoxy-1H-3-benzazepine

A solution of 3.8 g. N-(2,2-dimethoxyethyl)-3,4-dimethoxybenzeneethanimidamide hydrochloride in 15 ml. of trifluoroacetic acid was stirred for 5 hours at 50°C. The mixture stood overnight at room temperature and then was poured into water. A white precipitate formed. The solution was made basic with 25% sodium hydroxide, stirred for 10 minutes, and filtered to yield 2.0 g. of the title product, m.p. 170-172°C.

## Example 9

Alternate procedure for the preparation of 2-amino-7,8-dimethoxy-1H-3-benzazepine

A solution of 3.8 g. of N-(2,2-dimethoxyethyl)-3,4-dimethoxybenzeneethanimidamide hydrochloride in 10 ml. water and 10 ml. of concentrated sulfuric acid was stirred at room temperature for one hour. Sodium hydroxide (25%) was added to the mixture until the pH was about 13. The precipitate which formed was filtered and dried to yield 1.9 g. of the title product, m.p. 168-170°C.

CLAIMS

1. A process for preparing compounds of formula I

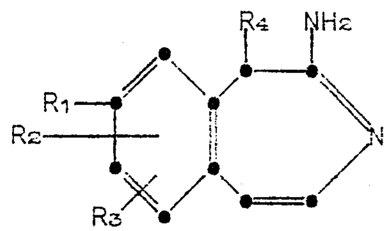

I

or pharmaceutically acceptable salts thereof, wherein:

$R_1$ is $C_1$-$C_3$ alkoxy;

$R_2$ and $R_3$ are each independently hydrogen, $C_1$-$C_3$ alkoxy, or $C_1$-$C_3$ alkyl; and

$R_4$ is hydrogen or methyl;

which comprises reacting a compound of formula II

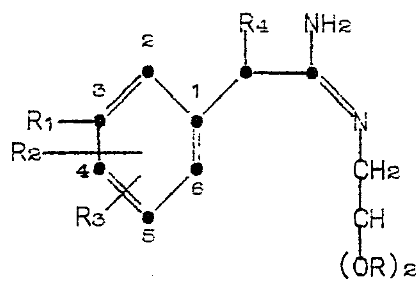

II

or salt thereof, wherein $R_1$, $R_2$, $R_3$ and $R_4$ are defined as above and R is $C_1$-$C_6$ alkyl, with at least a catalytic amount of one or more acids, optionally in the presence of one or more non-reactive solvents.

2. The process according to claim 1 wherein $R_4$ is hydrogen.

3. The process according to claim 1 or 2 wherein $R_3$ is hydrogen.

4. The process according to claim 1, 2 or 3 wherein $R_2$ is $C_1$-$C_3$ alkoxy.

5. The process according to claim 1, 2 or 3 wherein $R_1$ and $R_2$ are each methoxy.

6. The process according to claim 1 or 5 for preparing 2-amino-7,8-dimethoxy-1H-3-benzazepine.

7. The process according to claim 1 for preparing 2-amino-6,7,8-trimethoxy-1H-3-benzazepine.

8. The process according to claim 1 for preparing 2-amino-8-methoxy-1H-3-benzazepine.

9. The process according to claim 1 for preparing 2-amino-7-methoxy-8-ethoxy-1H-3-benzazepine.

10. The process according to claim 1, 6, 7, 8 or 9 wherein the pharmaceutically acceptable salt is the hydrochloride salt.

11. The process according to claim 1 wherein the acid is one or more of the mineral acids, alkanesulfonic acids, aromatic sulfonic acids or perfluoronated carboxylic acids, especially trifluoroacetic acid.

12. The process according to claim 11 wherein the acid is hydrogen chloride.

13. The process according to claim 11 wherein the acid is methanesulfonic acid.

14. The process according to claim 1 wherein the non-reactive solvent is water, $C_2$-$C_{20}$ alkanoic acid especially acetic acid, $C_3$-$C_{20}$ alkenoic acids, $C_3$-$C_6$ alkynoic acids, ketones such as acetone, esters such as

ethyl acetate, halides such as methylene chloride, hydrocarbons such as hexane or toluene, ethers especially tetrahydrofuran or 1,2-dimethoxyethane.

15. The process according to claim 12 or 14 wherein a mixture of acetic acid and 1,2-dimethoxyethane is employed as a solvent.

16. The process according to claim 12, 13 or 14 wherein acetic acid is employed as a solvent.

17. A compound of formula II

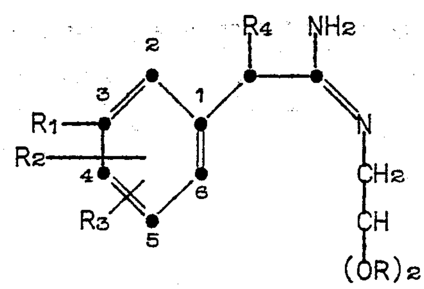

II

or salts thereof, wherein

$R_1$ is $C_1$-$C_3$ alkoxy;

$R_2$ and $R_3$ are each independently hydrogen, $C_1$-$C_3$ alkoxy, or $C_1$-$C_3$ alkyl;

$R_4$ is hydrogen or methyl; and

R is $C_1$-$C_6$ alkyl.

18. A compound of claim 17 wherein $R_4$ is hydrogen.

19. A compound of claim 17 or 18 wherein $R_3$ is hydrogen.

20. A compound of claim 17, 18 or 19 wherein $R_2$ is $C_1$-$C_3$ alkoxy.

21. The compound of claim 17 which is N-(2,2-dimethoxyethyl)-3,4-dimethoxybenzeneethanimidamide hydrochloride.

## CLAIMS

1. A process for preparing compounds of formula I

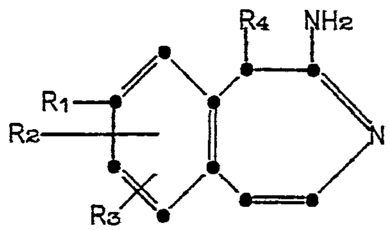

I

or pharmaceutically acceptable salts thereof, wherein:

$R_1$ is $C_1$-$C_3$ alkoxy;

$R_2$ and $R_3$ are each independently hydrogen, $C_1$-$C_3$ alkoxy, or $C_1$-$C_3$ alkyl; and

$R_4$ is hydrogen or methyl;

which comprises reacting a compound of formula II

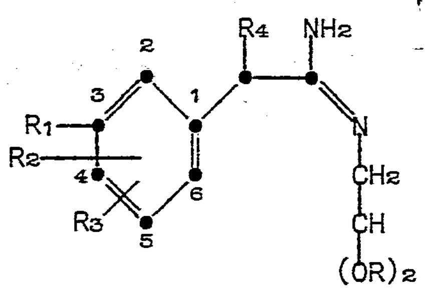

II

or salt thereof, wherein $R_1$, $R_2$, $R_3$ and $R_4$ are defined as above and R is $C_1$-$C_6$ alkyl, with at least a catalytic amount of one or more acids, optionally in the presence of one or more non-reactive solvents.

2. The process according to claim 1 wherein $R_4$ is hydrogen.

3. The process according to claim 1 or 2 wherein $R_3$ is hydrogen.

4. The process according to claim 1, 2 or 3 wherein $R_2$ is $C_1$-$C_3$ alkoxy.

5. The process according to claim 1, 2 or 3 wherein $R_1$ and $R_2$ are each methoxy.

6. The process according to claim 1 or 5 for preparing 2-amino-7,8-dimethoxy-1H-3-benzazepine.

7. The process according to claim 1 for preparing 2-amino-6,7,8-trimethoxy-1H-3-benzazepine.

8. The process according to claim 1 for preparing 2-amino-8-methoxy-1H-3-benzazepine.

9. The process according to claim 1 for preparing 2-amino-7-methoxy-8-ethoxy-1H-3-benzazepine.

10. The process according to claim 1, 6, 7, 8 or 9 wherein the pharmaceutically acceptable salt is the hydrochloride salt.

11. The process according to claim 1 wherein the acid is one or more of the mineral acids, alkanesulfonic acids, aromatic sulfonic acids or perfluoronated carboxylic acids, especially trifluoroacetic acid.

12. The process according to claim 11 wherein the acid is hydrogen chloride.

13. The process according to claim 11 wherein the acid is methanesulfonic acid.

14. The process according to claim 1 wherein the non-reactive solvent is water, $C_2$-$C_{20}$ alkanoic acid especially acetic acid, $C_3$-$C_{20}$ alkenoic acids, $C_3$-$C_6$ alkynoic acids, ketones such as acetone, esters such as

ethyl acetate, halides such as methylene chloride, hydrocarbons such as hexane or toluene, ethers especially tetrahydrofuran or 1,2-dimethoxyethane.

15. The process according to claim 12 or 14 wherein a mixture of acetic acid and 1,2-dimethoxyethane is employed as a solvent.

16. The process according to claim 12, 13 or 14 wherein acetic acid is employed as a solvent.